# EUROPEAN PATENT SPECIFICATION

(11) **EP 0 470 230 B1**
(45) Date of publication and mention of the grant of the patent: **04.06.1997**
(21) Application number: 91904924.7
(22) Date of filing: 13.02.1991
(51) Int. Cl.: C12N 15/54, C12N 9/12

(54) **METHOD FOR OBTAINING AND PURIFYING THE CARBOXYL-TERMINAL FRAGMENT OF THE DNA POLYMERASE I OF STREPTOCOCCUS PNEUNOMIAE**
VERFAHREN ZUR HERSTELLUNG UND REINIGUNG DES CARBOXYTERMINALEN FRAGMENTS DER DNA-POLYMERASE I VON STREPTOCOCCUS PNEUMONIAE
PROCEDE D'OBTENTION ET DE PURIFICATION DU FRAGMENT CARBOXYLE-TERMINAL DE L'ADN POLYMERASE I DU STEPTOCOCCUS PNEUMONIAE

(30) Priority: 23.02.1990 ES 9000542
(43) Date of publication of application: 12.02.1992
(73) Proprietor: CONSEJO SUPERIOR DE INVESTIGACIONES CIENTIFICAS, E-28006 Madrid (ES)
(72) Inventor: LOPEZ GARCIA, Paloma, Centro de Inves. Biologicas, Velazquez, 144, E-28006 Madrid (ES); PONS SALVADOR, M Elena, Centro de Inves.Biol.del, elasques, 144, 28006 Madrid (ES); DIAZ CARRASCO, Asuncion, Centro de Invest. Biol., Velasquez, 144, E-28006 Madrid (ES); ESPINOSA PADRON, Manuel, Centro de Invest. Biol., Velasquez, 144, E-28006 Madrid (ES); LACKS, Sanford, A., Upton, NY 11973 (US)
(74) Representative: Ungria Lopez, Javier
(86) International application number: ES9100010
(87) International publication number: WO9113153

(56) References cited:
- JOURNAL OF BIOLOGICAL CHEMISTRY, vol. 264, no. 7, 05 March 1989, US; P. LOPEZ et al., pp. 4255-4263
- PROCEEDINGS OF THE NATL. ACADEMY OF SCIENCES USA, vol. 80, April 1983, Washington, DC (US); C.M. JOYCE et al., pp. 1830-1834
- GENE, vol. 44, 1986, Elsevier Science Publishers B.V.; S. MARTINEZ et al., pp. 79-88
- CELL, vol. 59, 06 October 1989, Cell Press; A. BERNAD et al., pp. 219-228

## Description

Polymerases are used in Genetic Engineering techniques for the manipulation and characterization of genes whose products are of interest. In this aspect, the routine use in the laboratory of enzymes for the manipulation of DNA, has lead to hyperproduction and marketing of a large variety of them: DNA polymerase I (Pol I) of Escherichia coli (Minkley et al. (1984) J. Biol. Chem. 259: 10386-10392), the Klenow fragment of said PolI (Joyce and Grindley (1983) 80: 1830-1834), DNA polymerase of coliphage T4 (Lin et al. (1987) Proc. Natl. Acad. Sci. USA 84: 7000-7004) and DNA polymerase of Thermus aquaticus (Taq polymerase) (Peterson (1988) Nucl. Acids. Res. 22: 10915.) All of these enzymes have the characteristic of having an exonuclease activity, which is a problem for some of the Genetic Engineering techniques in which they are used. Recently, a chemical modification of DNA polymerase of coliphage T7 has been marketed, in which its exonuclease 3' → 5' reactivity has been eliminated (Tabor and Richardson (1987) Proc. Natl. Acad. Sci. USA 84: 4767-4771.)

We have cloned the gen polA of S. pneumoniae and we have hyperexpressed (Martinez et al. (1986) Gene 44: 79-88) and purified (López et al. (1989) J. Biol. Chem. 264: 4255-4263) its genic product PolI of said bacteria. Besides, this enzyme has polymerase activity, exonuclease 5' → 3' and 3' → 5' activities.

A method to subclone a fragment of the gene polA of S. pneumoniae, in the expression vector of E. coli. is described in the present patent application. By means of this process the recombinant plasmid pSM10 (Fig. 1) has been obtained. Said plasmid codifies for a polypeptide that has a DNA polymerase activity of the enzyme Pol I of S. pneumoniae. The protein has been hyperproduced in E. coli, the amount of active enzyme obtained corresponding to 7 % of the cellular proteinic content (Fig. 2.) The protein has been purified to homogeneity in said system (Fig. 3), having obtained 9,882 units from 500 ml of culture (Table I.) This invention has the advantage of obtaining a DNA polymerase without exonuclease activity with a high yield and low production cost. This fact may permit in the future industrial production thereof and the subsequent marketing thereof.

### DESCRIPTION OF THE DRAWINGS

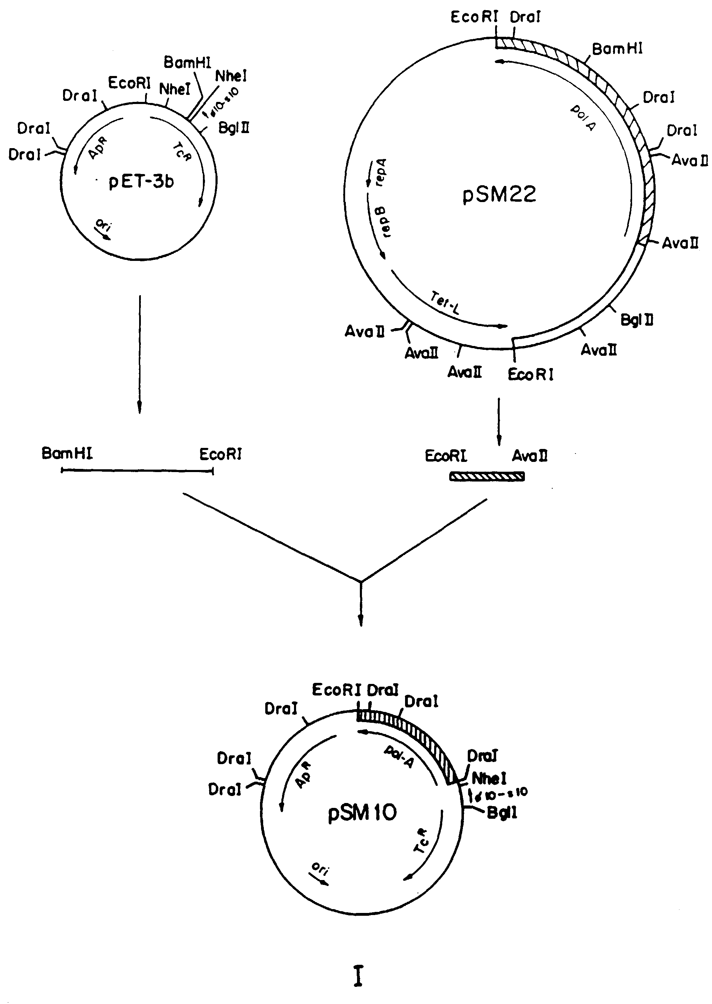

Figure 1. Construction of the plasmid pSM10 that contains the terminal fragment 3' of the gene polA of S. pneumoniae under the control of the promoter φ 10 of T7. It contains the restriction maps of the plasmids pET-3b, pSM22 and pSM10. The genetic symbols used are: polA, codifies for PolI of S. pneumoniae, Tc, tet-L and and Ap codify for the proteins responsible for the resistance to tetracycline and ampicillin respectively; repA and repB, codify for the proteins implied in the replication of the plasmid pSM22; ori, region of the origin of replication of the plasmids pET-3b abd psM10; φ 10 and S10, promoter and ribosomal binding site of the gene 10 of the coliphage. The arrows. indicate the direction of transcription.

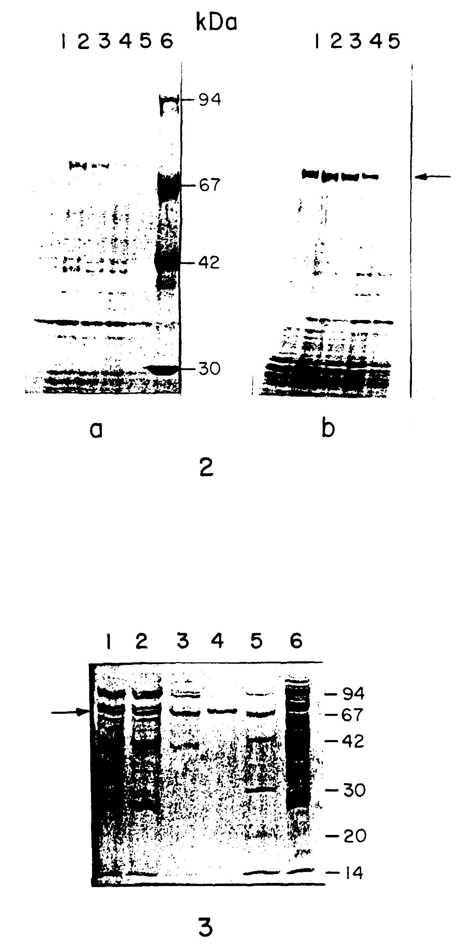

Figure 2. Hyperproduction of the carboxyl-terminal fragment of PolI in E. coli BL21DE3 (pLysS) (pSM10). (a) Gel dyed for proteins with coomassie blue. (b) autoradiography of the dry gel. Extracts prepared from cells incubated with IPTG for 30 min. followed by 60 min with rifamicine (path 1) and just with IPTG, 180 min. (path 2), 120 min. (path 3), 60 min. (path 4), 30 min. (path 5). Path 6 molecular weight standard.

Figure 3. Stages of purification of the carboxyl-terminal fragment of PolI. Paths: L: molecular weight standard, 2: total cellular extract, 3: ammonium sulfate precipitate, 4: fractions 44-49 of the filtration column in agarose, 5: fractions 23-29 of the heparin-agarose column, 6: fractions 27-29 of the DEAE-Sephacel column.

### EMBODIMENT OF THE INVENTION

### Example 1

Construction of the plasmid pSM10. The cloning was done in the strain of E. coli BL21DE3 (pLysS) (Studier et al.) Methods in Enzymol 185 : 60-89 (1989)). This bacteria contains in its chromosome the gene that codifies for the RNA polymerase of the coliphage T7 under the control of the promoter lac UV5. Its expression is inducible by isopropyl-β-thiogalactopyranoside (IPTG), this RNA polymerase, specifically recognizing the promoters of the bacteriophage T7. The plasmid pLysS codifies for the lysozyme of T7 this being a specific inhibitor of the RNA polymerase of T7. The vector used was pET-3b (Rosenberg et al. (1987) Gene 56: 125-135,) that contains the promoter and ribosornal binding site of the gene φ 10 of T7 and that makes it possible to effect a genic fusion in the site BamHI. Professor F.W. Studier of Brookhaven Nat. Lab. (USA Dept. of Energy) has patented in the USA the strain of E. coli BL21DE3, as well as the vector pET-3b.

The vector pET-3b was digested with the restriction enzymes BamHI and EcoRI. One of the fragments obtained BamHI-EcoRI of 4.1 kb of pET-3b was linked , in equimolecular proportions to the fragment EcoRI-AvaII of 1.8 kb of pSM22 (that contains part of the gen polA of S. pneumoniae) with DNA ligase of the phage T4. Once the ligation of the cohesive ends generated by the enzyme EcoRI has been carried out, the cohesive ends generated by AvaII and BamHI with the Klenow fragment of DNA polymerase I of E. coli are filled.Afterwards the linking of the blunt ends was done using DNA ligase of the phage T4. The linking mixture was used to transform E. coli BL21DE3 (pLysS). The ampicillin resistant transformants were analyzed for plasmid content in 1 % agarose gels. The recombinant plasmid obtained was called pSM10. The plasmid was analyzed bydigestion with restriction enzymes. The determination of the DNA nucleotide sequence showed that there were no rearrangements in the union area between the vector and the chromosomic insert, as a result the open reading area of the gene polA was in phase with that present in the vector.

### Example 2

### Hyperexpression of the carboxyl-terminal fragment of PolI.

Cells of the strain E. coli BL21DE3 (pLyS) (pSM10) were induced with 0.5 mM IPTG or IPTG and rifamicine 0.2 mg/ ml for different times (Fig. 2.) Crude extracts of the induced cultures were prepared (according to Martinez et al (1986) Gene 44: 70-88) and the protein content thereof was analyzed in SDS-polyacrylamide gels (linear gradient of 5% to 25% of polyacrylamide.) The strain containing pSM10 showed the presence of a protein of approximately 70 kDa, that increased with the induction time (Fig. 2 paths 5-1). The detected protein has a size corresponding to the one expected by genic fusion inferred from the coding DNA nucletide sequence (70,572 kDa).

### Example 3

Polymerase activity test. Cells containing pET-3b or pSM10 were induced for 3h with IPTG. Crude extracts were prepared and their polymerase activity was determined (according to Martinez et al. (1986) Gene 44: 79-88). The increase of approximately 100 times in the polymerizing activity of the strain E. coli BL21DE3 (pLysS) (pSM10) compared with that detected in the strain BL21DE3 (pLysS) (pET-3b) showed hyperexpression of the polypeptide with DNA polymerase activity and as part of this invention designated carboxyl-terminal fragment of DNA polymerase I of S. pneumoniae. Cultures of E. coli BL21DE3 (pLysS) (pSM10) were grown in a M9 medium containing 0.2 mg/ml of ampicillin up to OD₅₀₀ O.5. 0.5 mM IPTG was added and the incubation was continued for 3h. 1 ml samples were sedimented by centrifugation for 5 min. at 5,000 x g and resuspended in 0.15 ml of 10 mM TrisHCl pH 7.6 3 mM - mercaptoethanol, 0.1% trithon x 100. After 10 minutes incubation at 30°C, the cultures were broken by freezing at -70º C and defrosting at 37º C. The determination of the activity was done as we have described above.

### Example 4

### Purification of the carboxyl-terminal fragment of Pol I.

Step 1: Obtainment of cellular extracts. 500 ml of BL21DE3(pLysS)(pSM10) culture induced for 3 hours with IPTG (as described in example 3) were centrifuged 15 min at 5,000 x g and were concentrated 20 times in the buffer A (20 mM Tris-HCl) (pH 7.6), 1 mM DTT, 1 mM EDTA). The cellular extract was prepared by 3 cycles of freezing and defrosting at -70º C and at 37º C, respectively. All the subsequent steps of purification were carried out at 4º C. The elimination of the cellular debris was done by centrifugation at 15,000 x g for 30 min. The supernatant was designated crude extract (Table I.) Streptomycin sulfate at a final concentration of 5.8 % was added to supernatant and the nucleic acids were precipitated by incubation for 30 min and subsequent centrifugation at 15,000 x g 30 min. The precipitate was discarded and to the supernatant (designated streptomycin sulfate supernatant Table I) ammonimum sulfate at 65 % saturation was added. The resulting precipitate was designated ammonium sulfate precipitate (Table I) and was dissolved in 5 ml of buffer A (buffer A supplemented with 50 mM NaCl) and 5 % ethylene glycol).

### Step 2: Chromatography in agarose filtration column

The proteins contained in the ammonium sulfate precipitate were applied to an agarose filtration column (Bio-Rad Bio-Gel A-O, 5 m (85 x 1.6 cm) previously balanced with buffer B. The sample was eluted with the same buffer at 16.6 ml/h gathering 2 ml fractions.

The fractions were analyzed for polymerase activity and for protein content in 12 % SDS-polyacrylamide gels. A polymerase activity peak was detected in fractions 42-53. Fractions 43-50 which contained the maximum polymerizing activity and the minimum contamination to continue with the purification, were chosen.

### Step 3: Chromatography in heparin-agarose column

The peak of the agarose column was fractioned in a 10 ml. (15 x 0.9 cm) heparin-agarose column (Bio Rad) previously balanced with buffer B. The sample was eluted at 10 ml/h with a linear gradient of 100 ml of 50-500 mM NaCl in buffer B. The carboxyl-terminal fragment of Pol I of S. pneumoniae eluted as a peak in fractions 22-30, at a concentration of 100 mM NaCl. The fractions containing polymerase activity were combined on the basis of maximum activity and minimum contamination of proteins. These fractions (23-29) were diluted with buffer B without NaCl, to give a final concentration of 50 mM NaCl.

### Step 4: Chromatography in a DEAE-Sephacel column:

The peak of the Agarose-Heparin column was applied in a 10 ml. column (15 x 0.9 cm) of DEAE-Sephacel (Pharmacia) balanced with buffer B. The sample was eluted at 12 ml/ h with a linear gradient of 100 ml of 50-500 mM NaCl in buffer B. The pneumococcus polymerase appeared as a peak in fractions 26-31, eluting at a concentration of 200 mM NaCl. The fractions containing the enzyme were stored at -20º C in 50 % glycerol. In these conditions the enzyme is stable at least for 3 months.

The analysis of the different steps of the purification is gathered in figure 3 and on table I. The protein shows a degree of purity higher than 95 %. The yield corresponds to 0.73 mg of protein as of 500 ml of bacterial culture, with a specific activity of 13,472 units/ml. The recovery corresponds to 26.4 % of the polymerizing activity detected in crude extracts. The protein does not have exonuclease activity and it is free of contaminating nucleases. Less than 5 % of degradation of DNA marked with ³²P (in the 5' end or in the 3' end) was detected after 2 h. of incubation with 5 units of the enzyme. No degradation of the covalently closed or linearized plasmid DNA was observed after 20 h of incubation with 3 units of enzyme.

**Table I.**

| Purification of the carboxyl-terminal fragment of the DNA polymerase I of S. pneumoniae | | | |
|---|---|---|---|
| Sample | Proteins (mg) | Specific activity (u/mg) | Yield (%) |
| Crude extract | 87.45 | 427 | 100 |
| Streptomycin sulfate supernatant | 83.82 | 347 | 76.7 |
| Ammonium sulfate precipitate | 55.35 | 626 | 92.6 |
| Agarose fractions (44-49) | 9.81 | 260 | 68.2 |
| Heparin-agarose fractions (23-29) | 2.03 | 986 | 37.9 |
| DEAE-Sephacel fractions (27-29) | 0.73 | 13537 | 26.4 |

(a) 100 % corresponds to 37418 units of polymerase activity. One unit of polymerase activity is defined as the amount of enzyme that catalyzes the incorporation of 10 nmols of dNTP in 30 min. at 37º C.

The E. coli BL21DE (pSM10) (pLysS) strain containing the plasmid psM10 is deposited in the American Type Culture Collection, accession no. 68081.

## Claims

1. Method of obtainment and purification of the carboxyl-terminal fragment of DNA polymerase I of Streptococcus pneumoniae, characterized by the obtainment comprising:
a) construction of the recombinant plasmid pSM10 (contained in an E. coli BL21DE3 (pSM10) (pLysS) strain ATCC no. 68081) by means of obtainment of a fragment AvaII-EcoRI (1.8 kb) of the plasmid pSM22, which contains the terminal portion 3' of the gene polA of S. pneumoniae; insertion of said DNA fragment in the fragment BamHI-EcoRI (4.1 kb) of the vector plasmid pET-3b by means of linking; selection of transformed cells and obtainment of the plasmid;
b) analysis and determination of the DNA polymerase activity of the product of the plasmid pSM10, called carboxyl-terminal fragment of the PolI of S. pneumoniae.

2. Method according to claim 1 characterized by the purification of the carboxyl-terminal fragment of the PolI of S. pneumoniae comprising:
a) preparation of cellular extracts by means of growth of cultures of E. coli BL21DE3 (pSM10)(pLysS) in hyperexpression conditions of the enzyme; cellular lysis by freezing and defrosting; precipitation of the nucleic acids with streptomycin sulfate;
b) separation of proteins by means of differential precipitation with ammonium sulfate; fractioning of proteins by chromatography in columns of agarose, heparin-agarose and DEAE-Sephacel.

3. The pure carboxyl-terminal fragment of PolI of S. pneumoniae of 70.6 kDa which is the expression product of the plasmid pSM10 (ATCC no. 68081) a DNA polymerase lacking nuclease activity, capable of catalyzing the synthesis nuclease activity, capable of catalyzing the synthesis of DNA template, using 5-triphosphate deoxyribonucleosides as precursors.

## Patentansprüche

1. Verfahren zur Gewinnung und Reinigung des carboxyterminalen Fragments der DNA-Polymerase I von *Streptococcus pneumoniae,* dadurch gekennzeichnet, daß das Verfahren zur Gewinnung folgende Schritte enthält:
a) Konstruktion des rekombinanten Plasmids pSM10 (enthalten in einem *E. coli*-BL21DE3(p5M10)(pLysS)-Stamm, ATCC Nr. 68081), indem ein Fragment AvaII-EcoRI (1,8 kb) des Plasmids pSM22 hergestellt wird, das den terminalen 3'-Bereich des Gens polA von *S. pneumoniae* enthält; Insertion dieses DNA-Fragments in das Fragment BamHI-EcoRI (4,1 kb) des Vektorplasmids pET-3b durch Verknüfung; Selektion von transformierten Zellen und Gewinnung des Plasmids;
b) Analyse und Bestimmung der DNA-Polymerase-Aktivität des Produkts des Plasmids pSM10, genannt carboxyterminales Fragment des PolI von *S. pneumoniae.*

2. Verfahren gemäß Anspruch 1, dadurch gekennzeichnet, daß die Reinigung des carboxyterminalen Fragments des PolI von *S. pneumoniae* folgende Schritte enthält:
a) Präparation von zellulären Extrakten, indem Kulturen von *E. coli*-BL21DE3(pSM10)(pLysS) in Hyperexpressionsbedingungen für das Enyzm gezüchtet werden; zelluläre Lyse durch Einfrieren und Auftauen; Präzipitation der Nukleinsäuren mit Streptomycinsulfat;
b) Abtrennung von Proteinen durch differentielle Präzipitation mit Ammoniumsulfat; Fraktionierung der Proteine durch Chromatographie auf Agarose-, Heparin-Agarose- und DEAE-Sephacel-Säulen.

3. Das reine carboxyterminale Fragment von PolI von *S. pneumo*niae mit 70,6 kDa, das das Expressionsprodukt des Plasmids pSM10 (ATCC Nr. 68081) ist, eine DNA-Polymerase, die keine Nuklease-Aktivität aufweist, und die fähig ist, die Synthese von einer DNA-Matrize zu katalysieren unter Verwendung von 5-Triphosphat-deoxyribonukleosiden als Vorläufer.

## Revendications

1. Procédé d'obtention et de purification du fragment carboxyle terminal d'ADN polymérase I de Streptococcus pneumoniae, caractérisé en ce que l'obtention comprend :
a) la synthèse du plasmide recombiné pSM10 (contenu dans une souche de E. coli BL21DE3 (pSM10)(pLysS), ATCC n°68081) au moyen de l'obtention d'un fragment AvaII-EcoRI (1,8 kb) du plasmide pSM22 qui contient la portion terminale 3' du gène polA de S. pneumoniae; l'insertion dudit fragment d'ADN dans le fragment BamHI-EcoRI (4,1 kb) du plasmide vecteur pET-3b par liaison; la sélection de cellules transformées et l'obtention du plasmide ;
b) l'analyse et la détermination de l'activité ADN polymérase du produit du plasmide pSM10, appelé fragment carboxyle terminal de PolI de S. pneumoniae.

2. Procédé selon la revendication 1, caractérisé en ce que la purification du fragment carboxyle terminal de PolI de S. pneumoniae comprend :
a) la préparation d'extraits cellulaires par la réalisation de cultures de E. coli BL32DE3 (pSM10) (pLysS) dans des conditions d'hyperexpression de l'enzyme ; la lyse cellulaire par congélation et décongélation; la précipitation des acides nucléiques par le sulfate de streptomycine ;
b) la séparation de protéines par précipitation différentielle par du sulfate d'ammonium; le fractionnement de protéines par chromatographie sur des colonnes d'agarose, d'héparine-agarose et de DEAE-Sephacel.

3. Fragment carboxyle terminal pur de PolI de S. pneumoniae de 70,6 kDa, qui est le produit de l'expression du plasmide pSM10 (ATTC n° 68081), ADN polymérase dépourvue d'activité nucléase, capable de catalyser la synthèse de la matrice d'ADN, en utilisant des 5-triphosphate désoxyribonucléosides comme précurseurs.
